**Europäisches Patentamt**

(19) **European Patent Office**

**Office européen des brevets**

(11) Publication number: **0 125 520**

**A2**

(12) **EUROPEAN PATENT APPLICATION**

(21) Application number: **84104354.0**

(22) Date of filing: **17.04.84**

(51) Int. Cl.³: **C 07 D 301/32**

(30) Priority: **06.05.83 SE 8302611**

(43) Date of publication of application:
**21.11.84 Bulletin 84/47**

(84) Designated Contracting States:
**AT BE CH DE FR GB IT LI LU NL**

(71) Applicant: **Gambro Lundia AB**
**Box 10101**
**S-220 10 Lund(SE)**

(72) Inventor: **Jonsson, Ernst Uno**
**Örnvägen 43**
**S-222 31 Lund(SE)**

(72) Inventor: **Lindqvist, Sten-Börje**
**Plommongatan 3**
**S-240 14 Veberöd(SE)**

(72) Inventor: **Wimmerstedt, Roland Verner**
**Vallkärravägen 41**
**S-222 51 Lund(SE)**

(74) Representative: **Boberg, Nils Gunnar Erik**
**Gambro AB Patent Department Box 10101**
**S-220 10 Lund(SE)**

(54) A process and a plant for the recovery of one or more constituents from a gas mixture.

㉗ A process for the recovery of one or more constituents from a gas mixture containing ethylene oxide and the said constituent or constituents, the ethylene oxide being absorbed in water, whereupon the aqueous solution is removed from the remaining constituents which are less readily soluble in water.

The invention is designed in particular to be applied to a gas mixture intended for sterilization purposes comprising beside ethylene oxide gases such as carbon dioxide and/or chlorofluorocarbon which are relatively inert from a point of view of sterilization.

The invention also relates to a plant for the realization of processes of the abovementioned type. This plant here comprises means (3) for the bringing together of the gas mixture with liquid from a liquid source (6) for at least partial absorption of an undesirable constituent and means (4) for the separation of the non-absorbed part of the gas mixture, the said means (3-6) forming part of a recycling circuit which also comprises means (13-15) for the removal of the undesirable constituent.

TITLE

A PROCESS AND A PLANT FOR THE RECOVERY OF ONE OR MORE CONSTITUENTS FROM A GAS MIXTURE

TECHNICAL FIELD

The present invention relates to a process for the recovery of one or more constituents from a gas mixture containing ethylene oxide and the said constituent or constituents.

The process in accordance with the invention is designed in particular to be applied to a gas mixture intended for sterilization purposes comprising beside ethylene oxide gases such as carbon dioxide and/or chlorofluorocarbons which are relatively inert from a point of view of sterilization.

Ethylene oxide ($C_2H_4O$) can be used in pure form for the sterilization of heat-sensitive plastics. Preferably, however, it is used in mixture with one or more inert gases, such as carbon dioxide or different chlorofluorocarbons such as Freon 11 ($CFCl_3$) or Freon 12 ($CCl_2F_2$). In a number of the mixtures which are marketed the insert gas serves only as a complement so as to make the mixture non-flammable and thus also non-explosive in air.

An example of a customary sterilizing gas mixture is one of 12% by weight ethylene oxide and 88% by weight Freon 12. The reason for this composition is that ethylene oxide is non-flammable in air in a mixture with Freon 12, if the admixture of ethylene oxide is kept below approx. 14-15% by weight. Since ethylene oxide and Freon 12 cost approximately equally much to manufacture it would be desirable from a point of view of economy to recover both the components or at least the major component, Freon.

In accordance with the invention a plant for the recovery of one or more constituents from a gas mixture is also realized.

## BACKGROUND ART

It is known, for example from the US patent specification 4 249 917, that the abovementioned two components can be recovered simultaneously by dissolving them both in a common solvent.

Owing to the simultaneous solution of both components, great demands are made on the solvent.

According to US patent specification 4 112 054 the ethylene oxide is removed from a gas mixture instead with conversion at the same time to ethylene glycol and polyethylene glycol. In the case of this process too only very special solvents can be used.

Finally it is known that the gas mixture may be used again as such, but under accurate control of its composition. See for example US patent specification 3 372 980. In normal sterilization processes however, the gas mixture sooner or later becomes diluted with such an amount of air that it will no longer be safe. In such a situation the gas mixture must be either substituted or in some manner regenerated.

## DISCLOSURE OF INVENTION

The present invention is based instead on the principle known in itself that ethylene oxide is soluble in water. Compare, for example, US patent 4 221 727.

In accordance with the invention therefore the ethylene oxide is absorbed in water, whereupon the aqueous solution is removed from the remaining constituents which are less readily soluble in water.

Water here constitutes an inexpensive solvent which is also environmentally acceptable. To reduce further the cost of the process, however, it is appropriate to recycle the water after the ethylene oxide has been wholly or partly removed from the same.

The water is fed to the gas mixture appropriately in two stages. In a first stage the recycled water may be fed to the gas mixture so as to produce a coarse separation. The mixture of water and gas is then separated by means of free gas discharge from a separating tank with a free liquid surface. Subsequently more ethylene oxide is separated in that the partially cleaned gas is appropriately conducted in countercurrent through a water absorption tower. Water should be fed to this tower at one, two or more points. It is suitable to this end to feed clean water at a higher point and recycled water at one or more lower points or levels. Through this feed of clean water not only a very effective separation of ethylene oxide is brought about, but moreover a certain compensation for liquid losses in other parts of the system is achieved.

When the water used is recycled the ethylene oxide can be removed from the same through stripping by allowing the contaminated water to encounter a vigorous countercurrent of air in a desorption tower.

After the removal of the ethylene oxide the recovered constituent(s) can be compressed and/or cooled. This may be done in two or more stages.

Finally the recovered constituent(s) have to be dried which may be done either in gas phase or in liquid phase.

The invention also relates to a plant for the recovery of one or more constituents from a gas mixture preferably by means of the abovementioned process. This plant is characterized by means for the bringing together of the gas mixture with liquid from a liquid source for at least partial absorption of an undesirable constituent and by means for the separation of the non-absorbed part of the gas mixture, the said means forming part of a recycling circuit which also comprises means for the removal of the undesirable constituent.

The plant in accordance with the invention thus is intended in particular for the removal of ethylene oxide from a sterilizing gas mixture comprising ethylene oxide and gases such as carbon dioxide and/or chlorofluorocarbons which are relatively inert from a point of view of sterilization, e.g. Freon. In this application the plant is characterized in that the said liquid source is constituted of a water tank, a pump being adapted to pump water therefrom to a separating tank with simultaneous mixing and delivery of the gas mixture treated, the said separating tank comprising separate outlets for gas and liquid respectively.

Several sources of the gas mixture treated may be connected appropriately to a common point for simultaneous or successive treatment. These sources can be connected either directly to the point of the plant where a first mixing with liquid takes place or else via one or more pretreatment stages to one or more subsequent points. The water containing ethylene oxide obtained from the coarse separation may be collected, for example, in a joint collecting tank at the same time as the gas mixture remaining after the coarse separation is taken to a subsequent point in the system for further cleaning.

The gas outlet from one or more separating tanks which are adapted to bring about a coarse separation of the undesirable constituent may be connected, for example, to a lower point of a water absorption tower to which clean water is fed at an upper point and recycling water at one or more points or levels located in between.

The said means for the removal of the undesirable constituent from the recycling circuit appropriately consists of a cooling tower or stripping tower which is adapted so as to be vigorously blown through by means of a blower in countercurrent relatively to the liquid flow through the tower.

If two or more gas sources form part of the plant in accordance with the invention, it is equipped appropriately with an intermediate storage tank for the intermediate storage of liquids containing dissolved gas obtained from different sources. In this way a uniform loading is achieved so that no component has to be designed for the otherwise maximum possible load.

## BRIEF DESCRIPTION OF DRAWING

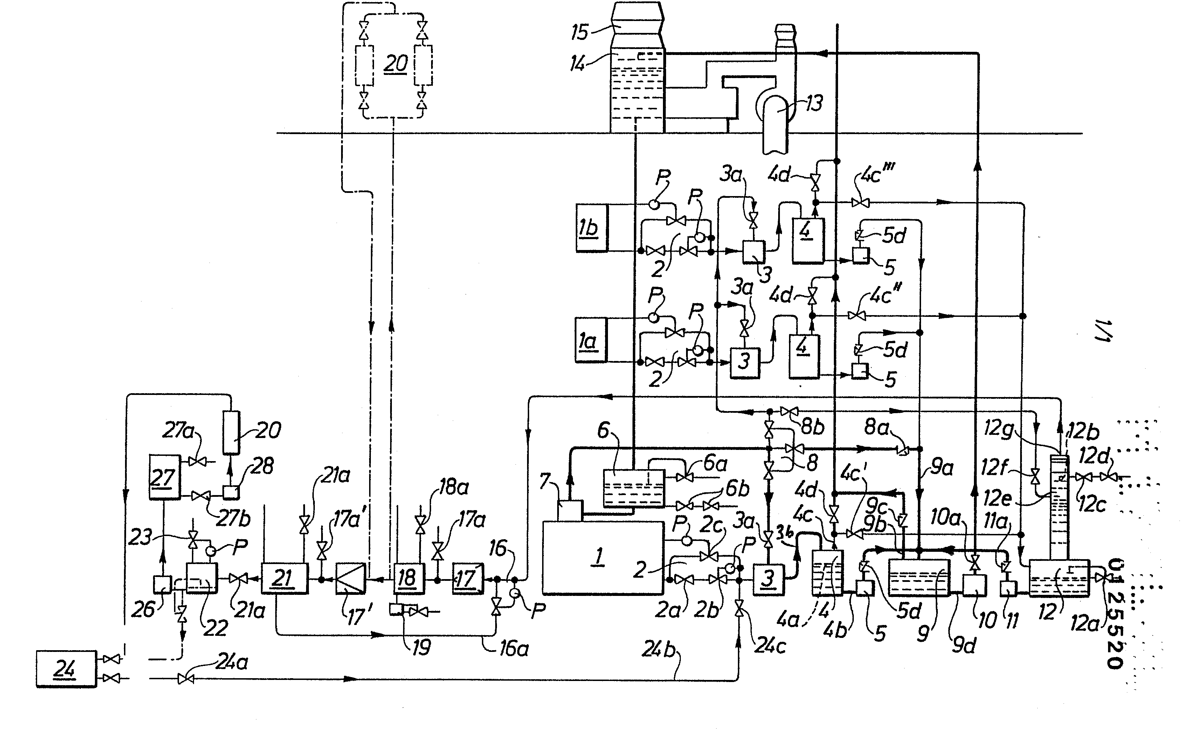

The invention will be described in more detail in the following with reference to the attached drawing which in the form of a block diagram shows a preferred embodiment of the subject of the invention and, in broken lines, an alternative embodiment.

## BEST MODE OF CARRYING OUT THE INVENTION

On the enclosed drawing a preferred embodiment of the subject of the invention is shown in full lines.

A first gas source, e.g. a gas sterilizing oven used, for example, for medical plastic articles which do not tolerate heat or radiation sterilization is designated 1.

A valve aggregate comprising three valves 2a, 2b and 2c and two pressure-detecting regulators P is designated 2. It is the object of this valve aggregate to reduce and thereby equalize the flow from the source 1, so that an even flow is obtained from this source during the greater part of the recovery cycle even though the pressure drops whilst the gas mixture is pumped from the source by means of the pump 3. This pump at the same time serves for the mixing of the gas mixture pumped out with the liquid which is delivered via a valve 3a and a valve system 8 from a liquid source 6. If it is ethylene oxide which is to be removed, this liquid will be water, to which may be added different additives however for special purposes. From the pump 3 the mixture of gas and liquid is pumped via the duct 3b to a separating tank 4 with a free liquid surface 4a. The tank comprises two outlets, namely a lower outlet 4b for liquid with gas dissolved therein and an upper outlet 4c for the non-absorbed gas constituent(s). These constituents subsequently are delivered further via a valve 4c' to a liquid absorption tower 12. Alternatively the gas mixture may be removed instead via a valve 4d. This is done, for example, in connection with the flushing of the gas sterilizing oven 1. This flushing takes place with the help of air and the flushed out gas becomes so diluted that it is unprofitable from a point of view of recovery. From the outlet 4b the liquid with the gas dissolved therein is pumped by means of a pump 5 via a non-return valve 5d to a collecting tank 9. To this tank clean or cleaned liquid can be delivered via a duct 9a from the liquid source 6 which provides the liquid pump 3 with liquid. The delivery of liquid to the tank 9 takes place in this case via the distributing valve system 8 and a regulating valve 8a. The tank 9 can also be vented via an outlet 9b and a non-return valve 9c to a chimney or the like. The liquid with gas absorbed therein delivered to the tank 9 is then passed via an outlet 9d by means of a pump 10 via a non-return valve 10a to a degassing installation 13-15 which will be described in greater detail in the following.

The gas separated in the separating tank 4 is thus delivered via the outlet 4c and the valve 4c' to a gas absorption tower 12. A level control means, schematically designated 12a, is arranged to increase or reduce the pumping of liquid from the tower 12 by means of a pump 11 via a non-return valve 11a to the tank 9. Liquid is fed to the tower 12 at an upper point or an upper level 12b. If ethylene oxide is to be removed this liquid is constituted of clean water which is fed via the valves 12c and 12d. At a lower point 12e further liquid is fed via a valve 12f. This liquid is taken appropriately from the liquid source 6 via the valve system 8 and a separate control valve 8b.

After cleaning in the gas absorption tower 12 the cleaned gas is delivered via an upper outlet 12g to a compression chamber 17. This is done via a pressure control means 16 which ensures an even flow through the chamber 16 by opening a gas return duct 16a if the pressure before the chamber 17 happens to be too low. In this way it is ensured that the tower 12 for example is not subjected to a harmful partial vacuum. The compressed gas is then delivered via a safety valve 17a to a heat exchanger 18 for the purpose of cooling. To this heat exchanger clean water is delivered via the valve 18a. Furthermore a liquid trap 19 is provided for the collection of any liquid precipitated from the gas in the heat exchanger.

Subsequently the gas is delivered to a further compression chamber 17' and from there via a further safety valve 17a' to a second heat exchanger 21 to which clean water is fed via the valve 21a. The recycling duct 16a leads away from a point inside the heat exchanger 21 where the gas has not yet been condensed.

The condensed gas is then passed from the heat exchanger 21 via a shutoff valve 21a to a storage tank 22 whose pressure is controlled by means of a pressure control device 23. With the help of a liquid pump 26 the gas is then pumped to a phase separator 27 which may be provided with coarse separating filters known in themselves, e.g. The DUSEC Coalescer from Knit Mesh Limited.

With the help of these filters a substantial part of the remaining water is separated via the valve 27a. The partially dried gas in turn is passed via the valve 27b with the help of a liquid pump 28 for final drying into a so-called molecular sieve 20. Such a sieve may be based on the fact for example that Freon has a molecular size 3 times that of water, so that water can be removed, with the help of a suitable drying medium of a pore size such that water can penetrate but not the Freon.

Finally the gas is delivered to gas holders 24. To make it possible to use the same gas holders by means of which the earlier gas mixture has been delivered, these gas holders can be evacuated via a valve 24a and a duct 24b with a further valve 24c with the help of the pump 3 before they are filled again with the cleaned part of the original gas mixture.

The designations 1a and 1b refer to further possible gas sources e.g. other sterilizing ovens which via their own valve systems 2 and pumps 3 together with coarse separation tanks 4 and further pumps 5 can be connected to the common collecting tank 9. At the same time the gas separated in the coarse separating tanks 4 can be delivered via valves 4c'' and 4c''' directly to the liquid absorption tower 12. In this manner the plant as a whole can be loaded uniformly without having to be designed for the theoretically possible maximum capacity.

From the collecting tank 9 the liquid with admixed gas is then conducted via the outlet 9d by means of the pump 10 and via the valve 10a to the gas separation installation 13-15. In the example shown this consists of a stripping tower 14 through which the liquid is passed in countercurrent relatively to the vigorous air flow produced with the help of a blower 13 as a result of which the gas absorbed in the liquid is degassed via a jet hood 15.  Alternatively, of course, this part of the original gas mixture may also be kept for further cleaning and reutilization.

Owing to the fact that liquid can be fed to the collecting tank not only from the separating tanks 4 but also directly from the liquid source 6, the degassing installation can operate the whole time under a substantially constant load.

Finally designations 6a and 6b refer respectively to a level control system 6a, with the help of which clean liquid can be delivered to the liquid source 6 and a draining valve 6b, with the help of which liquid can be continuously or intermittently discharged as required so as to prevent any excessive increase in the concentration of salts or the like owing to evaporation in the system, for example, in the gas separation installation 13-15.


ALTERNATIVE EMBODIMENT

An alternative embodiment is indicated in the drawing by broken lines.  In accordance with this the gas is dried in the gas phase with the help of two molecular sieves 20 connected in parallel before it is compressed in the compressor chamber 17' and cooled to liquid form in the heat exchanger 21.  In such an embodiment the storage tank 22 can be connected directly to the gas holder 24.

CLAIMS

1. A process for the recovery of one or more constituents from a gas mixture contaning ethylene oxide and the said constituent or constituents, c h a r a c t e r i z e d  in that the ethylene oxide is absorbed in water, whereupon the aqueous solution is removed from the remaining constituents which are less readily soluble in water.

2. A process in accordance with claim 1, c h a r a c t e r i z e d  in that it is applied to a gas mixture intended for sterilization purposes, comprising beside ethylene oxide gases such as carbon dioxide and/or chlorofluorocarbons which are relatively inert from a point of view of sterilization.

3. A process in accordance with anyone of the preceding claims, c h a r a c t e r i z e d  in that the water is recycled after the ethylene oxide has been wholly or partly removed from the same.

4. A process in accordance with anyone of the preceding claims, c h a r a c t e r i z e d  in that the water is fed in at least two stages (at 3, 12b and/ or 12e).

5. A process in accordance with anyone of claims 3 and 4, c h a r a c t e r i z e d  in that a coarse separation is brought about in that recirculated water is fed to the gas mixture in a first stage (at 3), whereupon the mixture of water and gas is separated through free gas discharge from a separating tank (4) with a free liquid surface (4a).

6. A process in accordance with claim 5, c h a r a c t e r i z e d  in that further ethylene oxide is separated in that the partially cleaned gas is passed in countercurrent through a water absorption tower (12).

7. A process in accordance with claim 6, characterized in that the water is fed to the tower (12) at two or more points (e.g. at 12b and 12e).

8. A process in accordance with claim 7, characterized in that clean water is fed to the tower at a higher point (12b) and recycled water at one or more lower points (e.g. 12e).

9. A process in accordance with anyone of claims 2-8, characterized in that ethylene oxide is removed from the water by vigorous blowing through of the water by means of air in a stripping installation (13-15).

10. A process in accordance with anyone of the preceding claims, characterized in that the recovered constituent(s) is (are) compressed and/or cooled (at 17 and 18).

11. A process in accordance with claim 10, characterized in that the compression and/or cooling is done in two or more stages (at 17 and 18 and at 17' and 21 respectively).

12. A process in accordance with anyone of the preceding claims, characterized in that the recovered constituent(s) is (are) dried (at 20 and/ or 27).

13. A process in accordance with claim 12, characterized in that the drying is carried out in the gas phase.

14. A process in accordance with claim 12, characterized in that the drying is carried out in the liquid phase.

15. A plant for the recovery of one or more constituents from a gas mixture preferably by means of a process according to anyone of the preceding claims, characterized by means (3) for the bringing together of the gas mixture with liquid from a liquid source (6) for at least partial absorption of a non- desirable constituent and by means (4) for the separation of the non-absorbed part of the gas mixture, the said means (3-6) forming part of a recycling circuit which comprises, moreover, means (13-15) for the removal of the undesirable constituent.

16. A plant in accordance with claim 15 for the removal of ethylene oxide from a sterilizing gas mixture comprising ethylene oxide and gases such as carbon dioxide and/or chlorofluorocarbons which are relatively inert from a point of view of sterilization, c h a r a c t e r i z e d   in that the said liquid source (6) is constituted of a water tank, a pump (3) being adapted to pump water therefrom to a separating tank (4) with simultaneous mixing and delivery of the gas mixture treated, the said separating tank (4) comprising separate outlets (4c and 4b respectively) for gas and liquid respectively.

17. A plant in accordance with anyone of claims 15 and 16, c h a r a c t e r i z e d   in that several sources (1, 1a, 1b) for the gas mixture treated are connected to a common point for simultaneous or successive treatment.

18. A plant in accordance with claim 16, c h a r a c t e r i z e d   in that the gas outlet (4c) from the separating tank (4) which is adapted to bring about a coarse separation of the undesirable constituent is connected to a lower point of a water absorption tower (12) to which clean water is fed at an upper point (12b) and recycling water at an intermediate point (12e).

19. A plant in accordance with claim 15, c h a r a c t e r i z e d   in that the said means (13-15) for the removal of the undesirable constituent from the recycling circuit consists of a cooling tower or stripping tower (14) which is adapted so as to be vigorously blown through by means of a blower (13) in countercurrent relatively to the liquid flow through the tower.

20. A plant in accordance with claim 17, c h a r a c t e r i z e d by an intermediate storage tank (9) for the intermediate storage of liquids containing absorbed gas obtained from different sources.

1/1

01255520